# EUROPÄISCHE PATENTSCHRIFT

(11) **EP 0 418 608 B1**
(45) Veröffentlichungstag und Bekanntmachung des Hinweises auf die Patenterteilung: **20.10.1993**
(21) Anmeldenummer: 90116616.5
(22) Anmeldetag: 30.08.1990
(51) Int. Cl.: B65D 65/32, A61F 13/02, G09F 3/02

(54) **Applikationshilfe und deren Verwendung**
Application means and its utilization
Moyen d'application et son utilisation

(30) Priorität: 16.09.1989 DE 3991019
(43) Veröffentlichungstag der Anmeldung: 27.03.1991
(73) Patentinhaber: LTS Lohmann Therapie-Systeme GmbH & Co. KG, 56513 Neuwied (DE)
(72) Erfinder: Hoffmann, Hans-Rainer, Dr., D-5450 Neuwied 22 (DE); Müller, Walter, Dr. Dipl.-Chem., D-5450 Neuwied 1 (DE); Kindel, Heinrich, D-5455 Rengsdorf (DE)
(74) Vertreter: Flaccus, Rolf-Dieter, Dr.

(56) Entgegenhaltungen:
- EP-A- 0 081 987
- EP-A- 0 284 963
- DE-C- 3 315 271
- GB-A- 2 179 910
- US-A- 3 230 649

## Beschreibung

Die Erfindung bezieht sich auf eine Applikationshilfe für einen oder mehrere auf einer flächenförmig sich erstreckenden, insbesondere flexiblen Träger- bzw. Abdeckschicht (1) haftend mechanisch lösbar angeordnete Gegenstände (2) wie therapeutische Systeme, Pflaster, Etiketten oder dergleichen in Form von in der Träger- bzw. Abdeckschicht (1) ausgebildeten Schnitten oder Sollbruchlinien (3), wobei jedem der Gegenstände (2) ein diese seitlich überragender Teil (4, 4a) der Träger- bzw. Abdeckschicht (1) zugeordnet ist, der mittels teilweise linearer Schnitte oder Sollbruchlinien (3) von der übrigen Träger- bzw. Abdeckschicht (1) abtrennbar ausgebildet ist, wobei die Schnitte oder Sollbruchlinien (3) zwei Punkte am Rande der Träger- bzw. Abdeckschicht (1) miteinander verbinden und mindestens teilweise unterhalb der Kontaktfläche der Gegenstände (2) verlaufen, mindestens einer dieser Punkte auf dem die Gegenstände (2) seitlich überragenden Teil (4,4a) der Träger- bzw. Abdeckschicht (1) liegt, und die gedachte gerade Verbindungslinie dieser beiden Punkte außer dem Anfangs- und Endpunkt keine weiteren gemeinsamen Punkte mit den Schnitten oder Sollbruchlinien (3) aufweist.

Es ist in vielerlei Art und Weise bekannt, Gegenstände haftend auf einer Unterlage anzuordnen und bei Bedarf diese abzulösen. Dies kann beispielsweise den Zweck haben, die Gegenstände leicht zugänglich zur Verfügung zu halten. Insbesondere wird von dieser Möglichkeit jedoch Gebrauch gemacht, wenn es darum geht, die in besonderer Weise präparierte Oberfläche von Gegenständen bis zur Anwendung gegen jedwede Beeinträchtigung zu schützen.

So ist es beispielsweise bekannt, die Haftklebeschicht von Etiketten, aber auch von Pflastern und anderen Gegenständen, mit einer abziehbaren Schutzfolie bis zum Zeitpunkt der Anwendung bzw. Benutzung abzudecken und zu schützen.

Das Ablösen der Gegenstände von der Trägerschicht bzw. das Abziehen der Abdeckschicht zur Freilegung der zu schützenden Fläche gestaltet sich insbesondere dann problematisch, wenn es sich um einen haftenden Verbund zwischen zwei Materialien handelt.
Dies trifft in ganz besonderem Maße dann zu, wenn die zu schützende Fläche des Gegenstandes sowohl während als auch nach ihrer Freilegung durch Ablösen von der Träger- bzw. Abdeckschicht vor Beschädigungen und/oder Kontamination bewahrt werden muß, beispielsweise um die Klebefähigkeit einer Klebeschicht, die durch mechanische Einflüsse nicht beeinträchtigte Unversehrtheit empfindlicher Oberflächen, eine Versiegelung der Fläche beispielsweise gegen Entweichen von in der Fläche angeordneten flüchtigen Komponenten usw. zu erhalten.

Insbesondere bei dem Versuch, die Partner mittels der Fingernägel, eines Messers oder eines anderen Instrumentes voneinander zu trennen, kommt es zu unerwünschten Berührungen oder gar Beschädigungen der zu schützenden Fläche und auch dazu, daß keine völlige Trennung der beiden Partner erzielt wird, vielmehr Teile der Träger- bzw. Abdeckschicht an der zu schützenden Oberflächen haften bleiben und diese dann nur sehr schwierig ohne Beeinträchtigung der schützenden Fläche entfernt werden können.

Ein solches Vorgehen verbietet sich vor allem dann, wenn es sich bei der zu schützenden Fläche um eine sterile Fläche von Verbänden, der Hautkontakschicht eines therapeutischen Pflasters oder in die Fläche eingearbeitete reaktive Materialien wie pharmazeutische Wirkstoffe handelt, die topisch verabreicht erst am Applikationsort mit der Haut in Kontakt treten dürfen, also beispielsweise Wirkstoffe, wie sie gemäß der deutschen Patentanmeldung P 39 01 551.3 zur Behandlung von aktinischen Keratosen in ein Pflastersystem eingearbeitet vorgeschlagen werden.

Zur Lösung der vorstehend aufgezeigten Problematik ist bereits vorgeschlagen worden, in der Träger- bzw. Abdeckschicht geradlinige Einschnitte oder Sollbruchlinien vorzusehen, so daß durch Biegen und/oder Abziehen der Träger- bzw.

Abdeckschicht Teile derselben vom haftend damit verbundenen Gegenstand losgelöst werden können. Eine einwandfreie vollständige und einfache Freilegung der Oberfläche des Gegenstandes bzw. eine entsprechende Ablösung desselben ist dabei jedoch nicht sichergestellt, insbesondere, wenn es sich um einen und empfindlichen Gegenstand handelt, wie beispielsweise um ein therapeutisches Pflaster oder ein Kunststofformteil oder eine sehr flexible Träger- bzw. Abdeckschicht aus einer dünnen Aluminiumfolie, einem Aluminiumlaminat, einer Polymerfolie oder dergleichen. Insbesondere bei kleinen Gegenständen mit einer kleinen zu schützenden Oberfläche ist es schwierig, diese völlig freizulegen. Aber auch das selektive Trennen einzelner Abschnitte von Gegenstand und Träger- bzw. Abdeckschicht ist nicht zufriedenstellend gelöst.

In der US-PS 3,230,649 ist das Anbringen von gekrümmten Einschnitten oder Sollbruchlinien in einer Trägerfolie beschrieben, die bei einem Biegen des Materials zur Bildung eines anfaßbaren Abschnittes des Trägermaterials führen, so daß dieser abgezogen werden kann. Diese Lösung stellt jedoch kein zufriedenstellendes Verfahren zum vollständigen und unbeschädigten Trennen der miteinander verbundenen Partner dar, denn in aller Regel muß ein verbleibender Rest der Trägerschicht mühsam mit Hilfe der Fingernägel oder mittels eines Hilfswerkzeuges vom Gegenstand entfernt werden.

Bekannt ist es auch, bei Anwendung von mehreren Gegenständen bzw. mehreren zusammengehörigen Teilen eines Gegenstandes diese mittels einer Art Ablöseband miteinander zu verbinden, so daß durch Ziehen an einem überstehenden Teil des Bandes die durch dieses miteinander verbundenen Teile in einem Arbeitsgang von der Trägerschicht abgezogen werden können. Vor Einsatz bzw. Anwendung der einzelnen Teile müssen diese jedoch noch vom Ablöseband getrennt werden.

Abgesehen von einer relativ aufwendigen Herstellung erlaubt diese bekannte Anordnung nur das gleichzeitige Ablösen von mehreren Gegenständen bzw. mehreren Teilen eines Gegenstandes und ist diese Anordnung auf verhältnismäßig kleindimensionierte Gegenstände bzw. Teile davon beschränkt.
Bekannt ist es, auch zwischen einer Trägerschicht und den auf diesen angeordneten Gegenständen Streifen oder Fäden aus einem geeigneten Material wie Kunststoff, Textil oder Metall vorzusehen, wobei diese mit wenigstens einem Ende über die Gegenstände bzw. Teile hinausragen, so daß mit ihrer Hilfe ein Trennen möglich ist. Diese Anordnung und Ausbildung ist jedoch mit einem sehr hohen Aufwand verbunden, ganz abgesehen davon, daß sie sich nicht in jedem Fall anwenden läßt.

In der europäischen Patentanmeldung EP-A 0 284 963 wird eine Abziehhilfe gemäß Figur 1 vorgeschlagen. Die Abziehhilfe ist gedacht für auf einem flächenförmigen flexiblen Trägermaterial (1.1) haftende, mechanisch ablösbare Substrate (1.2) und besteht aus nicht geradlinigen Schnitten und Sollbruchlinien (1.4) für jedes einzelne Substrat (1.2) im Trägermaterial (1.1). Durch Anwendung von Druck auf das Trägermaterial (1.1) mit einer Kraftkomponente senkrecht zu der Substratkontaktfläche ist ein durch die Sollbruchlinien oder Schnittlinien vorgegebener Teil des Substrates in Richtung des/der Substrats/e biegbar. Dadurch wird zumindest ein Teil des Substrats (1.2) in dessen Randbereich benachbart der Schnitt- oder Sollbruchlinie (1.4) vom Trägermaterial (1.1) abgelöst und bietet dadurch einen Anfaßschnitt (1.3) am Substrat (1.2) für das völlige Ablösen des Substrats (1.2) vom Trägermaterial (1.1).
Die vorgeschlagene Lösung des Problems erlaubt zwar das problemlose Ablösen von solchen klebenden Substraten, macht aber das Anfassen der klebenden Substratfläche mit den Fingern notwendig, so daß die Möglichkeit der Kontamination der Finger mit reaktiven Materialien des Subtrates gegeben ist.

Die GB-A-2 179 910 offenbart eine Applikationshilfe für einen oder mehrere auf einer flächenförmig sich erstreckenden, insbesondere flexiblen Träger- bzw. Abdeckschicht haftend mechanisch lösbar angeordnete Gegenstände wie beispielsweise ein Etikett oder dergleichen in der Form von in der Träger- bzw. Abdeckschicht ausgebildeten Schnitten oder Sollbruchlinien, wobei jedem der Gegenstände ein diese seitlich überragender Teil der Träger- bzw. Abdeckschicht zugeordnet ist, der mittels Schnitten oder Sollbruchlinien von der übrigen Träger- bzw. Abdeckschicht 3 abtrennbar ausgebildet ist, wobei die Schnitte oder Sollbruchlinien zwei Punkte am Rander der Träger- bzw. Abdeckschicht miteinander verbinden, mindestens teilweise unterhalb der Kontaktfläche der Gegenstände verlaufen, mindestens einer dieser Punkte auf dem die Gegenstände seitlich überragenden Teil der Träger- bzw. Abdeckschicht liegt, und die gedachte gerade Verbindungslinie dieser beiden Punkte außder dem Anfangs- und Endpunkt keine weiteren gemeinsamen Punkte mit den Schnitten oder Sollbruchlinien aufweist.
Das abzulösende Etikett besteht aus zwei Teilen, von denen der kleiner Teil mittels Schnitt- bzw. Sollbruchlinien vom ersten Teil abtrennbar ist. Bei der Lösung des kleineren Etiketteils von der nach Abtrennung vom größeren Etiketteil anhaftenden Abdeckschicht kommt es jedoch zu unerwünschten Berührungen oder gar Beschädigungen der zu schützenden Haftschicht unterhalb des abgetrennten Etiketteiles. Aufgrund dieses Nachteiles ergibt sich auch bei dieser bekannten Vorrichtung kein zufriedenstellendes Trennungsverfahren der miteinander verbundenen Teile, denn zur Applikation des kleineren Etiketteils muß der verbliebene Rest der Trägerschicht mit Hilfe der Fingernägel oder mittels eines Hilfswerkzeuges entfernt werden.

Der Erfindung liegt die Aufgabe zugrunde, eine Applikationshilfe der eingangs genannten Art zu schaffen, welche die vorstehend genannten Nachteile und Schwierigkeiten der bekannten Lösungen vermeidet und es gestattet, mit einer Träger- oder Abdeckschicht haftend aber mechanisch lösbar verbundene Gegenstände leicht und sicher sowie ohne Notwendigkeit bzw. Gefahr einer Kontaminierung oder Beschädigung der Kontakt- bzw. Schutzfläche von der Träger- bzw. Abdeckschicht abzulösen.

Diese Aufgabe wird gemäß der Erfindung mit einer Applikationshilfe nach Anspruch 1 gelöst. Zweckmäßige weitere Ausgestaltungen der Erfindung sind in den Unteransprüchen beschrieben.

Mit der erfindungsgemäßen Applikationshilfe wird erreicht, daß sowohl dann, wenn nur ein einziger Gegenstand oder aber auch deren mehrere mit einer Träger- bzw. Abdeckschicht haftend aber mechanisch lösbar verbunden sind, jeweils leicht und sicher ein genau vorbestimmter Teil der Träger- bzw. Abdeckschicht mit einem mit diesem haftend verbundenen Gegenstand von der Träger- bzw. Abdeckschicht abgetrennt wird, wobei gleichzeitig ein vorbestimmter Teil der geschützten Oberfläche des Gegenstandes freigelegt wird, ohne daß dabei die Notwendigkeit bzw. Gefahr einer Berührung mit der Hand gegeben ist, und sich der Gegenstand mit Hilfe des mit der Hand erfassten Teils der Träger- bzw. Abdeckschicht leich und bequem seiner weiteren Bestimmung zuführen lässt.

Die Erfindung ist in der Zeichnung in Ausführungsbeispielen gezeigt und wird anhand dieser im folgenden erläutert.

Es zeigen:
- Figur 1: eine quadratisch ausgebildete Träger- bzw. Abdeckschicht mit einem darauf angeordneten kreisrund ausgebildeten Gegenstand,
- Figur 2: einen von der Träger- bzw. Abdeckschicht der Figur 1 zusammen mit dem Gegenstand lösgelösten Abschnitt der Träger- bzw. Abdeckschicht,
- Figur 3: eine Variante zu Figur 1,
- Figur 4: eine weitere Variante zu Figur 1
- Figur 5: ein Ausführungsbeispiel für die Anordnung einer Mehrzahl von Gegenständen auf einer gemeinsamen Träger- bzw. Abdeckschicht.

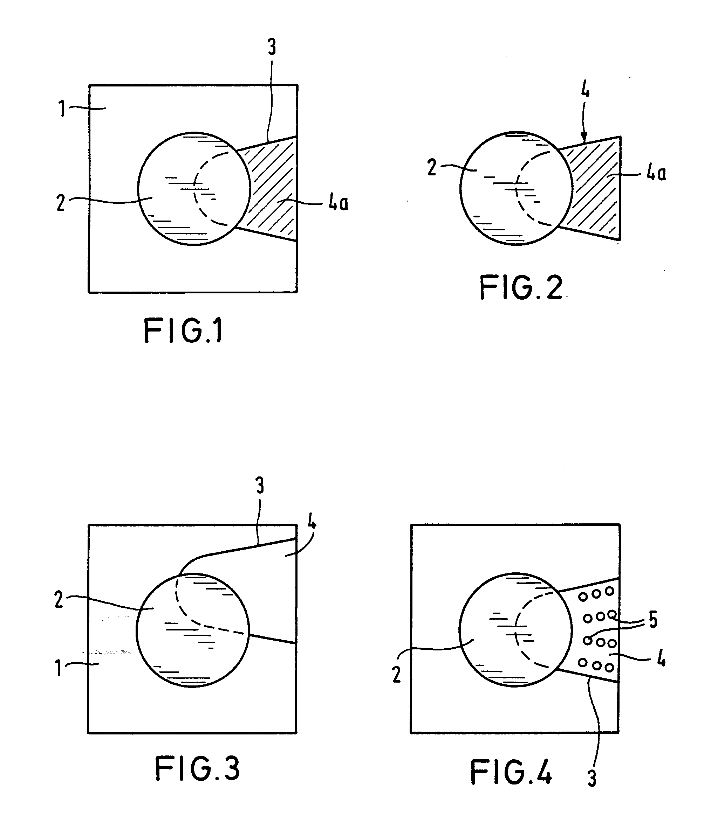

Nach Figur 1 und 2 ist auf der quadratisch ausgebildeten Träger- bzw. Abdeckschicht 1 mittig der mit kreisrundem Querschnitt gezeigte Gegenstand 2 angeordnet, wobei die Träger- bzw. Abdeckschicht 1 und der Gegenstand 2, bei dem es sich um ein Etikett, ein Pflaster, ein therapeutisches System oder dergleichen handeln kann, auf ihren einander berührenden bzw. gegenüberliegenden Kontaktflächen haftend aber mechanisch lösbar miteinander verbunden sein sollen.

In Bezug auf eine gedachte horizontale Mittellinie symmetrisch ist die Träger- bzw. Abdeckschicht 1 von ihrem rechten Rand ausgehend mit der im wesentlichen dreieckig ausgebildeten Schnitte oder Sollbruchlinie 3 versehen. Diese Schnitte oder Sollbruchlinie 3 kann beispielsweise eine über einen Teil der Dicke der Träger- bzw. Abdeckschicht sich erstreckende Einkerbung oder auch eine Perforierung mit einer mehr oder weniger großen Anzahl von mehr oder weniger dicht nebeneinander angeordneten Perforationslöchern mehr oder weniger großen Querschnitts sein. Ebenso könnte diese Schnitte oder Sollbruchlinie aber auch in Form einer mehr oder weniger großen Anzahl von durch Stege miteinander verbundenen mehr oder weniger langen Einschnitten ausgebildet sein. Hierbei wird selbstverständlich darauf geachtet, daß die mechanische Stabilität der Träger- bzw. Abdeckschicht 1 genügend groß ist, so daß ein unbeabsichtigtes und vorzeitiges Abtrennen - etwa beim Verpackungsvorgang - sicher vermieden ist.

Je nachdem, wie empfindlich die zu schützende Kontaktfläche des Gegenstandes 2 ist, kann auch vorgesehen werden, die Schnitte oder Sollbruchlinie 3 in dem Bereich, in dem sie sich unterhalb des Gegenstandes 2 erstreckt, als durch die Dicke der Träger- bzw. Abdeckschicht 1 nicht völlig hindurchgehende Einkerbung, in den sich außerhalb des Gegenstandes 2 erstreckenden Bereichen dagegen als Perforation bzw. Einschnitte mit dazwischen befindlichen Stegen ausgebildet sein. In jedem Fall wird die Ausbildung so getroffen, daß ein sicherer Schutz der zu schützenden Oberfläche des Gegenstandes 2 gewährleistet ist, daß sich aber der durch die Schnitte oder Sollbruchlinie 3 definierte Teil 4 der Träger- bzw. Abdeckschicht 1 leicht und sicher in der vorbestimmten Form und Weise vom Rest der Träger- bzw. Abdeckschicht 1 abtrennen läßt, wobei selbstverständlich auch dafür Sorge getragen ist, daß die Fläche, auf der sich der Teil 4 und der Gegenstand 2 haftend berühren, so gewählt ist, daß das einwandfreie Ablösen des Gegenstandes 2 von der Träger- bzw. Abdeckschicht ebenso sichergestellt ist, wie das sichere Haftenbleiben des Gegenstandes 2 am Teil 4 bei dessen Abtrennen vom Rest der Träger- bzw. Abdeckschicht.

Wie insbesondere aus Figur 2 deutlich zu ersehen ist, ist der schraffierte Bereich 4a des Teils 4 genügend groß ausgebildet, daß er ein sicheres Erfassen zwecks Abtrennen vom übrigen Teil der Träger- bzw. Abdeckschicht 1 erlaubt.

Anstatt einer quadratischen Ausbildung der Träger- bzw. Abdeckschicht 1 kann selbstverständlich auch jede andere gewünschte Form realisiert werden, wie auch der Gegenstand 2 selbstverständlich nicht nur kreisrunden Querschnitt, sondern auch jede andere denkbare Form aufweisen kann, je nachdem, um was für einen Gegenstand es sich im Einzelfall handelt. Desgleichen kann selbstverständlich auch die Form des Teils 4 völlig anders gestaltet sein, wie es sich eben als zweckmäßig oder auch erforderlich erweist. Schließlich ist auch die mittige Anordnung des Gegenstandes 2 auf der Träger- bzw. Abdeckschicht 1 hier nur beispielhaft zu verstehen und könnte auch eine irgendwie geartete außermittige bzw. asymmetrische Anordnung von Gegenstand 2 wie auch der Anordnung und Ausbildung des Teils 4 vorgesehen werden.

Eine solche Möglichkeit der asymmetrischen Anordnung des Teils 4 in Bezug auf die Träger- bzw. Abdeckschicht 1 und insbesondere den Gegenstand 2 ist in Figur 3 gezeigt, bei der die Schnitte oder Sollbruchlinie 3 am rechten Rand nach oben versetzt angeordnet ist, so daß der Teil 4 den Gegenstand 2 auf einem asymmetrisch geformten Flächenbereich haftend berührt.

Diese asymmetrische Anordnung und Ausbildung kann von Fall zu Fall geeignet sein, das Anwenden bzw. Applizieren der Gegenstände 2 zu erleichtern, wenn der Gegenstand 2 mittels des einen Teil der Fläche des Gegenstandes 2 bedeckenden und zu einem Teil 4a den Gegenstand 2 überragenden mitabgetrennten Teils 4 zu dem Applikationsort verbracht und dort ohne Berührung der Kontaktfläche des Gegenstandes auf dem Applikationsort appliziert wird. Dazu wird der Gegenstand zunächst mit dem freiliegenden Teil auf dem Applikationsort verankert, dann der abgetrennte Teil 4 der Träger- bzw. Abdeckschicht 1 entfernt und schließlich der Gegenstand vollflächig mit dem Applikationsort in Kontakt gebracht. Dabei muß zu keinem Zeitpunkt die wirkstoffhaltig oder in anderer Weise präparierte Fläche des Gegenstandes mit den Fingern oder einem sonstigen Hilfsmittel wie beispielsweise einer Pinzette oder dergleichen berührt werden.

Zwecks Erleichterung des Abtrenn- bzw. Ablösevorgangs ist gemäß Figur 4 der zum Anfassen mit der Hand bestimmte Bereich 4a des Teils 4 mit den Noppen 5 versehen, wodurch insbesondere ein sicheres Festhalten gewährleistet ist. Anstelle von Noppen könnten selbstverständlich auch Riffelungen oder dergleichen Erhebungen vorgesehen werden.

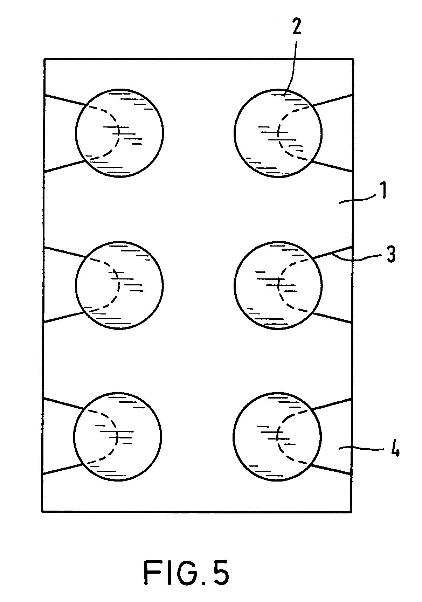

Ein Beispiel für die Möglichkeit der Anordnung und Aufbringung von mehreren Gegenständen 2 oder auch von Teilen eines Gegenstandes auf einer gemeinsamen Träger- bzw. Abdeckschicht 1 zeigt Figur 5, wobei selbstverständlich auch hier gilt, daß die symmetrische Ausbildung von Träger- und Abdeckschicht 1 sowie die Anordnung und Ausbildung der Gegenstände 2 und der Schnitte oder Sollbruchlinien 3 bzw. der durch diese gebildeten Abreißteile 4 bzw. Anfaßteile 4a nicht zwingend ist, vielmehr jede andere geeignete und gewünschte Ausbildung ohne Nachteile realisiert werden kann.

## Patentansprüche

1. Applikationshilfe für einen oder mehrere auf einer flächenförmig sich erstreckenden, insbesondere flexiblen Träger- bzw. Abdeckschicht (1) haftend mechanisch lösbar angeordnete Gegenstände (2) wie therapeutische Systeme, Pflaster, Etiketten oder dergleichen in Form von in der Träger- bzw. Abdeckschicht (1) ausgebildeten Schnitten oder Sollbruchlinien (3), wobei jedem der Gegenstände (2) ein diese seitlich überragender Teil (4, 4a) der Träger- bzw. Abdeckschicht (1) zugeordnet ist, der mittels teilweise linearer Schnitte oder Sollbruchlinien (3) von der übrigen Träger- bzw. Abdeckschicht (1) abtrennbar ausgebildet ist, wobei die Schnitte oder Sollbruchlinien (3) zwei Punkte am Rande der Träger- bzw. Abdeckschicht (1) miteinander verbinden und mindestens teilweise unterhalb der Kontaktfläche der Gegenstände (2) verlaufen, mindestens einer dieser Punkte auf dem die Gegenstände (2) seitlich überragenden Teil (4,4a) der Träger- bzw. Abdeckschicht (1) liegt, und die gedachte gerade Verbindungslinie dieser beiden Punkte außer dem Anfangs- und Endpunkt keine weiteren gemeinsamen Punkte mit den Schnitten oder Sollbruchlinien (3) aufweist, dadurch gekennzeichnet, daß der den Gegenstand (2) überdeckende Bereich des abtrennbaren Teils (4) der Träger- bzw. Abdeckschicht (1) asymmetrisch zum Gegenstand (2) ausgebildet ist.

2. Applikationshilfe nach Anspruch 1, dadurch gekennzeichnet, daß der die Gegenstände (2) überragende Teil (4) der Träger- bzw. Abdeckschicht (1) mit Noppen, Riffelungen oder dergleichen (5) versehen ist.

3. Applikationshilfe nach Anspruch 1 oder 2, dadurch gekennzeichnet, daß die Schnitte oder Sollbruchlinien (3) in Form einer mehr oder minder großen Anzahl von durch Stege miteinander verbundenen mehr oder weniger langen Einschnitten ausgebildet sind.

4. Applikationshilfe nach einem der Ansprüche 1 bis 3, dadurch gekennzeichnet, daß der abtrennbare Teil (4) der Träger- bzw. Abdeckschicht (1) mit dem restlichen Teil der Träger- bzw. Abdeckschicht (1) über wenige Verbindungsstege verbunden ist.

5. Applikationshilfe nach einem der Ansprüche 1 bis 4, dadurch gekennzeichnet, daß der den Gegenstand (2) überdeckende Bereich (4a) des abtrennbaren Teils (4) der Träger- bzw. Abdeckschicht (1) asymmetrisch zum Gegenstand (2) ausgebildet ist.

6. Verwendung der Applikationshilfe nach einem der vorhergehenden Ansprüche für flächenförmige therapeutische Systeme, Pflaster oder Etiketten.

## Claims

1. An application aid for one or several articles (2), such as therapeutic systems, plasters, labels, or the like, adhering to and positioned in a mechanically releasable manner on a carrier or covering layer (1), respectively, which extends in sheet-like form and particularly is flexible, in the form of cuts or predetermined breaking lines formed within the carrier or covering layer (1), whereby each article (2) is assigned a portion (4, 4a) of the carrier or cover layer (1), which portion laterally projects said article and is removable from the rest of the carrier or cover layer (1) by means of partially linear cuts or predetermined breaking lines (3), whereby the cuts or predetermined breaking lines (3) connect two points at the edge of the carrier or cover layer (1) and at least partially run below the contact surface of the articles (2), at least one of these points lies on that portion (4, 4a) of the carrier or covering layer (1) projecting the articles (2) at the side, and the imaginary straight connecting line between these two points, except for the starting and the final point, does not exhibit any other points of intersection with the cuts or predetermined breaking lines (3), characterized in that the region (4a) of the separable part (4) of the carrier or covering layer (1), which region overlaps the article (2), is formed asymmetrically with respect to the article (2).

2. An application aid according to claim 1, characterized in that the portion (4) of the carrier or covering layer (1) laterally projecting the articles (2) is provided with knops, engravings, or the like.

3. An application aid according to claim 1 or 2, characterized in that the cuts or predetermined breaking lines (3) are formed by a greater or lesser number of incisions of greater or lesser length which are connected by webs.

4. An application aid according to any one of claims 1 to 3, characterized in that the separable part (4) of the carrier or cover layer (1) is connected to the remaining part of the carrier or cover layer (1) via a few connecting webs.

5. An application aid according to any one of claims 1 to 4, characterized in that the region (4a) of the separable part (4) of the carrier or covering layer (1), which region overlaps the article (2), is formed asymmetrically with respect to the article (2).

6. The use of the application aid according to any one of the preceding claims in sheet-like therapeutic systems, plasters or labels.

## Revendications

1. Accessoire d'application pour un ou plusieurs objets (2) tels que systèmes thérapeutiques, pansements, étiquettes ou similaires, disposés de manière à adhérer sur une couche porteuse et de recouvrement (1) s'étendant superficiellement et en particulier flexible, et détachables mécaniquement de la couche porteuse et de recouvrement, le dispositif présentant la forme d'entailles ou de lignes de future rupture (3) formées dans la couche porteuse et de recouvrement (1), à chacun des objets (2) étant associée une partie (4, 4a), débordant latéralement de cet objet, de la couche porteuse et de recouvrement (1), cette partie étant réalisée de manière à être détachable du reste de la couche porteuse et de recouvrement (1) grâce à des entailles ou de lignes de future rupture (3) partiellement rectilignes, les entailles ou lignes de future rupture (3) reliant l'un à l'autre deux points situés sur le bord de la couche porteuse et de recouvrement (1) et s'étendant au moins partiellement au-dessous de la surface de contact des objets (2), au moins un de ces points étant situé dans la partie (4, 4a) de la couche porteuse et de recouvrement (1) qui déborde latéralement des objets (2), et la ligne droite imaginaire reliant ces deux points ne présentant en dehors de son point initial et de son point final aucun autre point commun avec les entailles ou lignes de future rupture (3), caractérisé en ce que la zone de la partie séparable (4) de la couche porteuse et de recouvrement (1) recouvrant l'objet (2) possède une forme asymétrique par rapport à l'objet (2).

2. Accessoire d'application selon la revendication 1, caractérisé en ce que la partie (4) de la couche porteuse et de recouvrement (1) débordant des objets (2) est dotée de boutons en relief, de cannelures ou similaires (5).

3. Accessoire d'application selon la revendication 1 ou 2, caractérisé en ce que les entailles ou lignes de future rupture (3) présentent la forme d'un nombre plus ou moins important d'entailles plus ou moins longues séparées l'une de l'autre par des passages.

4. Accessoire d'application selon l'une des revendications 1 à 3, caractérisé en ce que la partie séparable (4) de la couche porteuse et de recouvrement (1) est reliée au reste de la couche porteuse et de recouvrement (1) par un petit nombre de passages de liaison.

5. Accessoire d'application selon l'une des revendications 1 à 4, caractérisé en ce que la zone (4a), recouvrant l'objet (2), de la partie détachable (4) de la couche porteuse et de recouvrement (1) présente une forme asymétrique par rapport à l'objet (2).

6. Utilisation de l'accessoire d'application selon l'une des revendications précédentes pour des systèmes thérapeutiques, des pansements ou des étiquettes de forme plate.
